# EUROPEAN PATENT SPECIFICATION

(11) **EP 2 682 996 B1**
(45) Date of publication and mention of the grant of the patent: **27.07.2016**
(21) Application number: 12752772.9
(22) Date of filing: 16.02.2012
(51) Int. Cl.: H01L 51/46, H01L 51/42, C07C 333/16, C08G 79/00

(54) **THIN FILM SOLAR CELL**
DÜNNSCHICHTSOLARZELLE
PHOTOPILE EN COUCHES MINCES

(30) Priority: 03.03.2011 JP 2011046203
(43) Date of publication of application: 08.01.2014
(73) Proprietor: Japan Science and Technology Agency, Kawaguchi-shi, Saitama 332-0012 (JP)
(72) Inventor: OKUBO TAKASHI, Osaka 577-8502 (JP); KIM KYUNG HO, Osaka 577-8502 (JP); INUZUKA YOSHIE, Osaka 577-8502 (JP); TANAKA NAOYA, Osaka 577-8502 (JP); MAEKAWA MASAHIKO, Osaka 577-8502 (JP); KURODA TAKAYOSHI, Osaka 577-8502 (JP)
(74) Representative: Cabinet Plasseraud
(86) International application number: PCT/JP2012/053613
(87) International publication number: WO 2012/117860

(56) References cited:
- WO-A1-2011/148900
- WO-A2-2010/144472
- US-A- 5 698 048
- TAKASHI OKUBO ET AL: "Crystal structure and carrier transport properties of a new 3D mixed-valence Cu(i)-Cu(ii) coordination polymer including pyrrolidine dithiocarbamate ligand", DALTON TRANSACTIONS, vol. 40, no. 10, 1 January 2011 (2011-01-01), page 2218, XP055126042, ISSN: 1477-9226, DOI: 10.1039/c0dt01065k
- TAKASHI OKUBO ET AL: "Intervalence Charge-Transfer System by 1D Assembly of New Mixed-Valence Octanuclear Cu I /Cu II /Cu III Cluster Units", INORGANIC CHEMISTRY, vol. 50, no. 7, 28 February 2011 (2011-02-28), pages 2708-2710, XP055126048, ISSN: 0020-1669, DOI: 10.1021/ic101794u
- TAKESHI YAMADA ET AL.: 'Dithiocarbamate Yudotai o Kakyo Haiishi to shita Kongo Genshika Haii Kobunshi no Gosei to Taiyo Denchi eno Oyo' 90TH ANNUAL MEETING OF THE CHEMICAL SOCIETY OF JAPAN IN SPRING KOEN YOKOSHU II 12 March 2010, page 564
- KYUNG HO KIM ET AL.: 'Dye-sensitized Solar Cells with Halide-bridged Mixed-valence Cu(I)-Cu(II) Coordination Polymers with Hexamethylenedithiocarbamate Ligand' CHEMISTRY LETTERS vol. 39, no. 7, 2010, pages 792 - 793
- LLABRES I XAMENA, F. X. ET AL.: 'Applications for Metal-Organic Frameworks(MOFs) as Quantum Dot Semiconductors' JOURNAL OF PHYSICAL CHEMISTRY C vol. 111, no. ISS.1, 06 December 2006, pages 80 - 85

## Description

### Technical Field

The present invention relates to a thin-film solar cell, and more specifically, to a thin-film solar cell including a coordination polymer.

### Background Art

In recent years, sunlight has attracted attention as a source of green energy, and solar cells that use sunlight have been actively developed. Examples of solar cells include silicon solar cells, solar cells that use inorganic compounds, and thin-film solar cells typified by a bulk heterojunction thin-film solar cell that uses organic compounds.

Among these various types of solar cells, thin-film solar cells are solar cells having a structure in which a p-type organic semiconductor and an n-type organic semiconductor are formed into a thin film. Since the structure of thin-film solar cells is simple, the production cost is low. In addition, a sealing technique used in organic electroluminescence (EL) devices can also be used. Thus, in recent years, expectation of the practical use of thin-film solar cells as new-type solar cells has been rapidly increasing.

Among the thin-film solar cells, as described in NPL 1 and NPL 2, bulk heterojunction thin-film solar cells in which 1-(3-methoxycarbonyl)propyl-1-phenyl-[6,6]-C61 (PCBM), poly-3-hexylthiophene (P3HT), and the like are used as organic semiconductors are widely known.

In recent years, various technical developments for improving the conversion efficiency have been conducted. For example, PTL 1 discloses a bulk heterojunction thin-film solar cell whose conversion efficiency is improved by mixing inorganic nanoparticles together with organic semiconductors. Other examples and embodiments of thin-film solar cells comprising inorganic material mixed with an organic semiconductor can be found in PTL2.

### Citation List

### Patent Literature

PTL 1: Japanese Unexamined Patent Application Publication No. 2009-158730 PTL2: International Patent Application Publication No: WO 2010/144472

### Non Patent Literature

NPL 1: S. E. Shaheen, C. J, Brabec, N. S. Sariciftic, F. Padinger, T. Framherz, and J. C. Hummelen, Appl. Phys. Lett., Vol. 78, 2001, pp. 841-843 NPL 2: Y. Kim, S. Cook, S. M. Tuladhar, S. A. Choulis, J. Nelson, J. R. Durrant, D. D. C. Bradley, M. Giles, I. Mcculloch, C. Ha, and M. Ree, Nature, Materials, Vol. 5, 2006, pp. 197-203

### Summary of Invention

### Technical Problem

However, common thin-film solar cells disclosed in NPL 1 and NPL 2 have low conversion efficiencies, namely, 2.5% and 4.4%, and thus have a drawback in that these solar cells are inadequate for practical use.

In the bulk heterojunction thin-film solar cell described in PTL 1, the conversion efficiency is improved as follows: light taken into a cell is scattered by the inorganic nanoparticles, whereby the light taken into the cell is effectively used as much as possible without the light being emitted to the outside of the cell.

However, in this case, the following problems occur: Since the inorganic nanoparticles are not involved in light absorption, when the amount of inorganic nanoparticles added is excessively large, a ratio of the presence of an organic semiconductor necessary for absorbing visible light in the cell decreases. In addition, it becomes more difficult for light to be taken into the cell, or the transmission of generated photocarriers may be inhibited. Because of these various factors, the conversion efficiency is decreased instead, and thus there is a limit in the improvement in the conversion efficiency.

The present invention has been made in view of the above problems in the related art. An object of the present invention is to provide a thin-film solar cell in which a coordination polymer used absorbs light and efficiently supplies generated electrons and holes to organic semiconductors or electrodes, thereby increasing the charge separation efficiency and improving the conversion efficiency.

### Solution to Problem

To achieve the above object, a thin-film solar cell according to Claim 1 of the present invention includes at least one organic semiconductor; and a coordination polymer, in which the coordination polymer contains a repeating unit which includes a complex produced by coordinating at least one ligand to at least one metal ion, the metal ion being selected from ions of transition metal elements, and the ligand being capable of coordinating to the metal ion and selected from sulfur-containing compounds, nitrogen-containing compounds, oxygen-containing compounds, and phosphorus-containing compounds, the coordination polymer being disposed so as to be in contact with a p-type organic semiconductor and an n-type organic semiconductor and to form an interface between the p-type organic semiconductor and the n-type organic semiconductor, and when two or more organic semiconductors are used as the organic semiconductor, the two or more organic semiconductors form a bulk-hetero structure. The ligand is at least one derivative selected from derivatives of a dithiocarbamate ion, the derivatives being represented by Chem. 1 and Chem. 2 below: 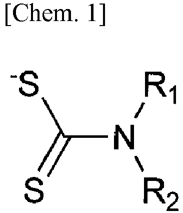 where R₁ and R₂ may be the same or different and each represent an aliphatic hydrocarbon group, a substituted aliphatic hydrocarbon group, an aromatic hydrocarbon group, a substituted aromatic hydrocarbon group, a heterocyclic group, or a substituted heterocyclic group, 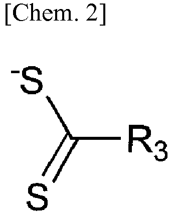 where R₃ represents a heterocyclic group or substituted heterocyclic group having at least one nitrogen atom.

In a thin-film solar cell according to Claim 2 of the present invention, the transition metal element is at least one metal element selected from Fe, Co, Ni, Cu, Ru, Rh, Pd, Ag, Os, Ir, Pt, and Au.

In a thin-film solar cell according to Claim 3 of the present invention, the coordination polymer further contains a bromine ion or an iodine ion.

In a thin-film solar cell according to Claim 4 of the present invention, the organic semiconductor is at least one substance selected from fullerene, fullerene derivatives, oligothiophene, oligothiophene derivatives, polythiophene, polythiophene derivatives, phthalocyanine, phthalocyanine derivatives, metal phthalocyanine, metal phthalocyanine derivatives, polyphenylene, polyphenylene derivatives, polyphenylene vinylene, polyphenylene vinylene derivatives, polyvinylcarbazole, polyvinylcarbazole derivatives, polysilane, polysilane derivatives, polyfluorene, polyfluorene derivatives, pentacene, pentacene derivatives, anthracene, anthracene derivatives, rubrene, rubrene derivatives, perylene, perylene derivatives, tetracyanoquinodimethane, tetracyanoquinodimethane derivatives, tetrathiafulvalene, tetrathiafulvalene derivatives, oxadiazole, and oxadiazole derivatives.

In a thin-film solar cell according to Claim 5 of the present invention, the organic semiconductor is 1-(3-methoxycarbonyl)propyl-1-phenyl-[6,6]-C61 (PCBM) and poly-3-hexylthiophene (P3HT), and the coordination polymer contains copper as the transition metal element, piperidinedithiocarbamic acid as the ligand, and an iodine ion.

First, a thin-film solar cell of the present invention will be described below.

The thin-film solar cell of the present invention includes a coordination polymer together with an organic semiconductor. Furthermore, the thin-film solar cell of the present invention includes, as an essential component, a coordination polymer having a repeating unit which includes a complex produced by coordinating at least one ligand to at least one metal ion, the metal ion being selected from ions of transition metal elements, and the ligand being capable of coordinating to the metal ion and selected from sulfur-containing compounds, nitrogen-containing compounds, oxygen-containing compounds, and phosphorus-containing compounds.

Herein, the term "coordination polymer" in the present invention refers to a polymer having a structure in which metal complexes are connected to each other, the metal complexes each including a metal ion located at the center and ligands composed of an organic compound and bonded to the periphery of the metal ion. Schematic examples thereof include a coordination polymer having a one-dimensional structure illustrated in Fig. 14, a coordination polymer having a two-dimensional structure illustrated in Fig. 15, and a coordination polymer having a three-dimensional structure illustrated in Fig. 16.

Examples of the coordination polymer include not only a coordination polymer having a repeating structure in which metal ions located at the center of respective metal complexes are cross-linked by a ligand, but also a coordination polymer containing a cross-linking agent component, such as a bromine ion or an iodine ion described below, other than a ligand, so as to cross-link units of a metal complex as required, and having a repeating structure in which cross-link is also formed by the other cross-linking agent component.

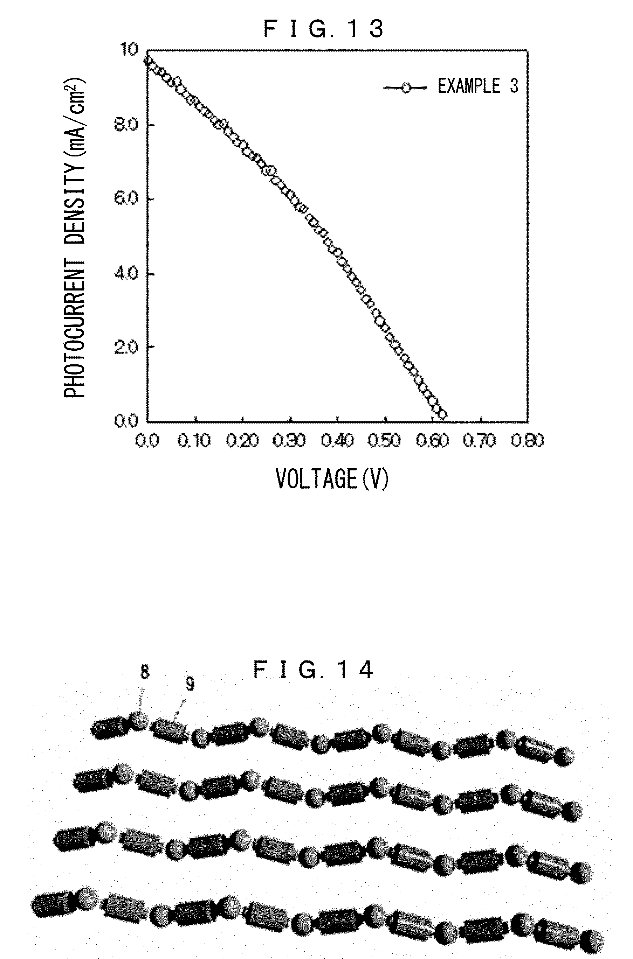

In the case where a coordination polymer skeleton is positively charged or negatively charged, in order to cancel the electric charges, an anion or a cation such as a ferrocenium ion or an ammonium ion may be incorporated in the coordination polymer skeleton typified by the structures illustrated in Figs. 14, 15, and 16. Such compounds are also covered by the coordination polymer.

Examples of requirements for a coordination polymer necessary for the thin-film solar cell of the present invention include the following: The coordination polymer is to exhibit high absorption in the visible light region, the coordination polymer is to have a capability of transporting generated electrons and holes, the energy level of the lowest unoccupied molecular orbital (LUMO) of the coordination polymer is to be higher than the LUMO of an organic n-type semiconductor or the Fermi level of a negative electrode, and the energy level of the highest occupied molecular orbital (HOMO) of the coordination polymer is to be lower than the HOMO of an organic p-type semiconductor or the Fermi level of a positive electrode.

Next, components constituting the coordination polymer will be described.

As the metal ion, metal ions of at least one element selected from transition metal elements are used in the present invention. Among transition metal elements, metal ions of elements of the so-called group 8 and group 1B such as Fe, Co, Ni, Cu, Ru, Rh, Pd, Ag, Os, Ir, Pt, and Au are preferably used. Furthermore, among these, a copper ion is preferably used from the standpoint that electrons can be delocalized over the entire coordination polymer by coordinating a ligand described below, and the cost is low.

Regarding these metal ions, by using not only one metal ion but also different types of metal ions in combination, characteristics of respective metal ions can be exhibited. For example, by using a metal ion having a heavy-atom effect, such as a platinum ion or an iridium ion, in combination with a copper ion, it is possible to exhibit not only the electron delocalization effect achieved by the copper ion but also the effect of extending the excitation lifetime, the effect being achieved by the platinum ion, the iridium ion, or the like.

As the ligand, at least one selected from sulfur-containing compounds, nitrogen-containing compounds, oxygen-containing compounds, and phosphorus-containing compounds that can coordinate to the above metal ion is used in the present invention. Examples of the sulfur-containing compounds include ligands having a dithiooxalate substituent, a tetrathiooxalate substituent, or a dithiocarboxylic acid substituent. Examples of the nitrogen-containing compounds include ligands having a pyrazine skeleton, a bipyridine skeleton, or an imidazole skeleton. Examples of the oxygen-containing compounds include oxalates, chloranilates, 2,5-dihydroxy-1,4-benzoquinone, benzene dicarboxylate, and catechol. Examples of the phosphorus-containing compounds include triphenylphosphine, diphenylphosphino ethane, diphenylphosphino propane, and 1,1'-bis(diphenylphosphino)ferrocene.

Among these compounds, derivatives of a dithiocarbamate ion, the derivatives being represented by Chem. 1 and Chem. 2 below, are used. 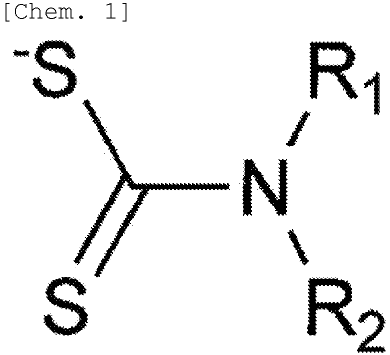 (R₁ and R₂ may be the same or different and each represent an aliphatic hydrocarbon group, a substituted aliphatic hydrocarbon group, an aromatic hydrocarbon group, a substituted aromatic hydrocarbon group, a heterocyclic group, or a substituted heterocyclic group.)

R₁ and R₂ each preferably have 1 to 50 carbon atoms and more preferably have 1 to 30 carbon atoms. 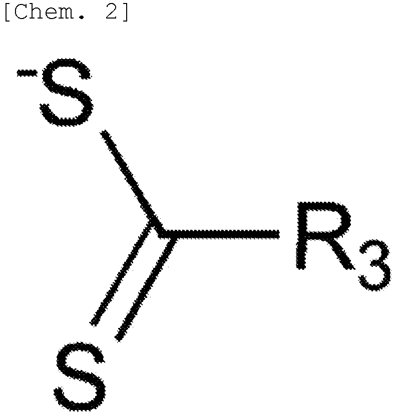 (R₃ represents a heterocyclic group or substituted heterocyclic group having at least one nitrogen atom.)

Specific examples of R₃ include heterocyclic groups and substituted heterocyclic groups represented by Chem. 3 below. Examples of R₃ that can be used further include groups in which groups that may be the same or different and that are each selected from aliphatic hydrocarbon groups, substituted aliphatic hydrocarbon groups, aromatic hydrocarbon groups, substituted aromatic hydrocarbon groups, heterocyclic groups, and substituted heterocyclic groups are bonded to any of the heterocyclic groups and substituted heterocyclic groups represented by Chem. 3.

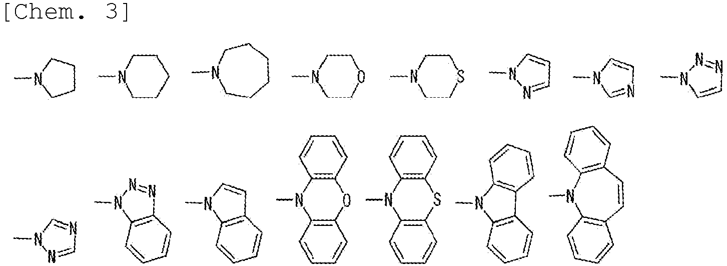

The coordination polymer of the present invention may include, besides the metal ion and the ligand, a halogen element as a cross-linking agent component for cross-linking the metal ions. Among halogen elements, from the standpoint of improving the electron delocalization effect, a bromine ion or an iodine ion is preferably used.

By using this cross-linking agent component, energy bands are formed by the highest occupied molecular orbital (HOMO) and the lowest unoccupied molecular orbital (LUMO) of each of the components. Consequently, the transporting properties of the generated electrons and holes can be improved.

For example, as illustrated in Chem. 4, a mononuclear complex in which hexamethylenedithiocarbamic acid functioning as a ligand is coordinated to a copper ion functioning as a metal ion is cross-linked by using a bromine ion or an iodine ion. With this structure, an energy band is formed by overlapping of orbitals, and thus a high carrier transporting property can be realized. 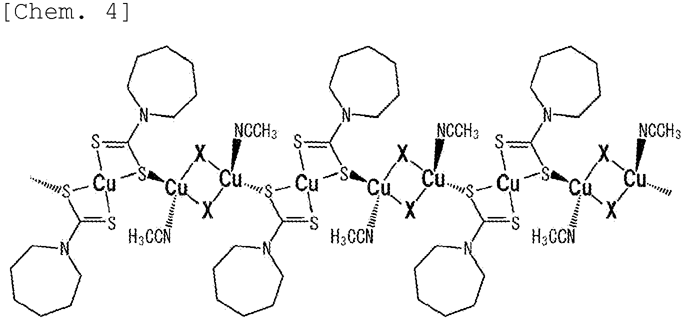 (X represents Br or I.)

The molecular weight and the number (n) of repeating units of the coordination polymer of the present invention are not particularly limited, and are appropriately determined in accordance with the organic semiconductor used. The molecular weight is preferably 1,000 or more, and the number of repeating units is preferably 100 or more. The upper limit of the molecular weight and the upper limit of the number (n) of repeating units can be appropriately determined within a range in which problems do not occur in the use of the coordination polymer.

The mixing ratio of the coordination polymer of the present invention to the organic semiconductor is also not particularly limited, and is appropriately determined in accordance with the organic semiconductor used. The mixing ratio of the coordination polymer is preferably 1% by weight or more relative to the amount of organic semiconductor.

The reason for this is that when the mixing ratio is less than 1% by weight, the effect due to the coordination polymer may not be sufficiently achieved.

The coordination polymer of the present invention can be produced by a known method, for example, by mixing compounds used as raw materials in an organic solvent. In the case where a bromine ion or an iodine ion is used as a cross-linking agent component, the coordination polymer can be produced by preparing a mononuclear complex, and then mixing the mononuclear complex with a bromine ionic compound or an iodine ionic compound. Specifically, for example, such a coordination polymer is produced by preparing a mononuclear complex in which hexamethylenedithiocarbamic acid is coordinated to a copper ion using a copper salt and hexamethylenedithiocarbamic acid, and then mixing the prepared mononuclear complex with copper bromide in an organic solvent.

An organic semiconductor used in the present invention functions as an n-type semiconductor or a p-type organic semiconductor. Compounds that have been used in thin-film solar cells can be used as the organic semiconductor.

Examples of the compounds include fullerene, fullerene derivatives, oligothiophene, oligothiophene derivatives, polythiophene, polythiophene derivatives, phthalocyanine, phthalocyanine derivatives, metal phthalocyanine, metal phthalocyanine derivatives, polyphenylene, polyphenylene derivatives, polyphenylene vinylene, polyphenylene vinylene derivatives, polyvinylcarbazole, polyvinylcarbazole derivatives, polysilane, polysilane derivatives, polyfluorene, polyfluorene derivatives, pentacene, pentacene derivatives, anthracene, anthracene derivatives, rubrene, rubrene derivatives, perylene, perylene derivatives, tetracyanoquinodimethane, tetracyanoquinodimethane derivatives, tetrathiafulvalene, tetrathiafulvalene derivatives, oxadiazole, and oxadiazole derivatives. Among these compounds, 1-(3-methoxycarbonyl)propyl-1-phenyl-[6,6]-C61 (PCBM) and poly-3-hexylthiophene (P3HT) are preferably used because a higher conversion efficiency can be realized.

In the case where the coordination polymer used in the present invention functions as an n-type semiconductor or a p-type organic semiconductor, only one of the above organic semiconductors may be used.

### Advantageous Effects of Invention

According to the thin-film solar cell of the present invention, a thin-film solar cell having a high conversion efficiency can be obtained at a low cost by using a coordination polymer which is a metal complex.

Among the ligands, when a derivative of a dithiocarbamate ion is used, the coordination polymer also functions as a sensitizing dye and thus a coordination polymer that efficiently absorbs visible light can be obtained.

The reason for this is as follows: Since the ionization energy of a transition metal element and the ionization energy of a derivative of a dithiocarbamate ion are close to each other, photoexcitation of the transition metal element, which is originally a forbidden transition, becomes allowed by hybridization of the orbital of the metal ion and the orbital of the ligand. As a result, the resulting coordination polymer including the derivative of a dithiocarbamate ion can efficiently absorb visible light as a whole. In addition, since an energy band is formed by the hybridization of the orbitals, the carrier transporting property is increased. As a result, electrical conductivity is exhibited.

In addition, by using a coordination polymer including a derivative of a dithiocarbamic acid, a metal ion of a transition metal element, and a bromine ion or an iodine ion, a thin-film solar cell having a higher conversion efficiency can be obtained.

Furthermore, when a coordination polymer including piperidinedithiocarbamic acid, a copper ion, and an iodine ion is used, the energy level of the HOMO of the coordination polymer can be made lower than the HOMO level of a p-type semiconductor (in particular, P3HT), and the energy level of the LUMO of the coordination polymer can be made higher than the LUMO level of an n-type semiconductor (in particular, PCBM). Thus, electrons and holes generated by charge separation on the coordination polymer can be efficiently supplied to the organic semiconductors, and a thin-film solar cell having an even higher conversion efficiency can be obtained.

### Brief Description of Drawings

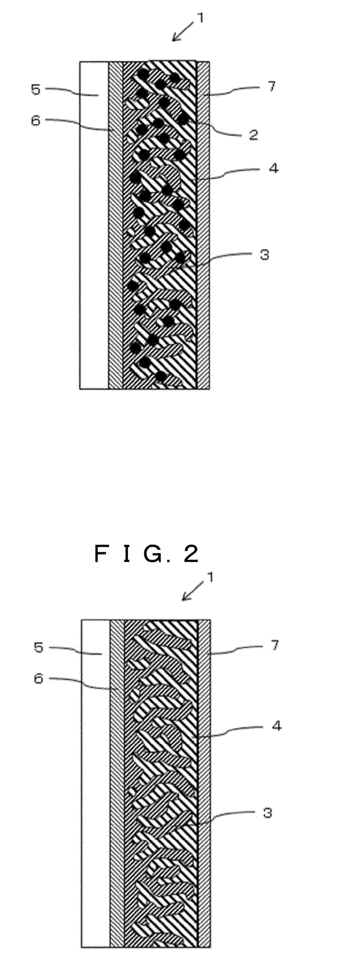

[Fig. 1] Fig. 1 is a schematic view illustrating an example of a cross-sectional structure of a thin-film solar cell according to the present invention.
[Fig. 2] Fig. 2 is a schematic view illustrating a cross-sectional structure of an existing thin-film solar cell.

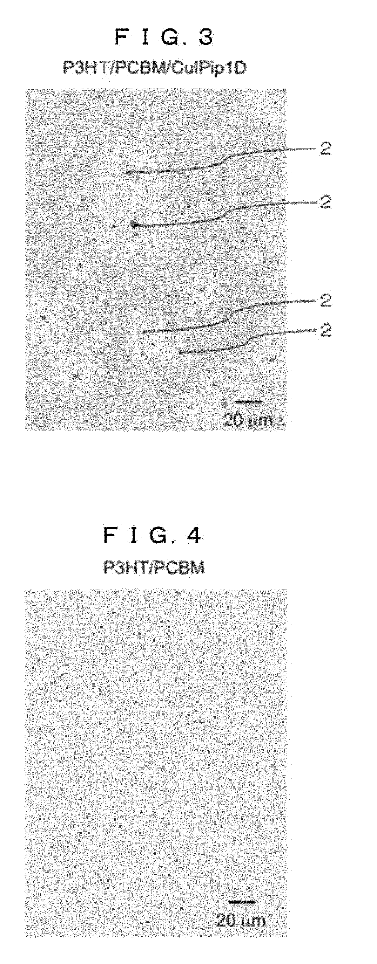

[Fig. 3] Fig. 3 is a laser microscope photograph of an organic layer of a thin-film solar cell according to the present invention.
[Fig. 4] Fig. 4 is a laser microscope photograph of an organic layer of an existing thin-film solar cell.

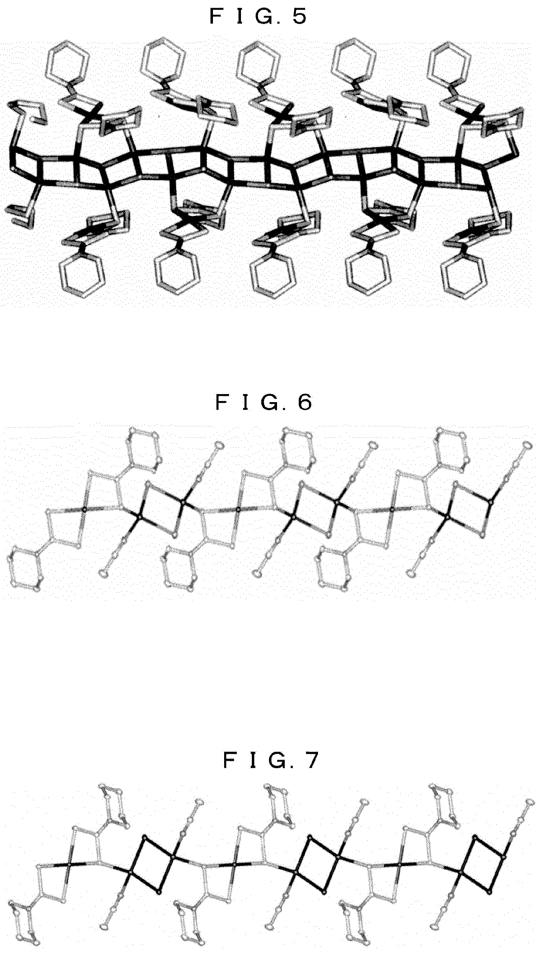

[Fig. 5] Fig. 5 is a schematic view illustrating a three-dimensional structure of a coordination polymer of Production Example 8.
[Fig. 6] Fig. 6 is a schematic view illustrating a three-dimensional structure of a coordination polymer of Production Example 16.
[Fig. 7] Fig. 7 is a schematic view illustrating a three-dimensional structure of a coordination polymer of Production Example 17.

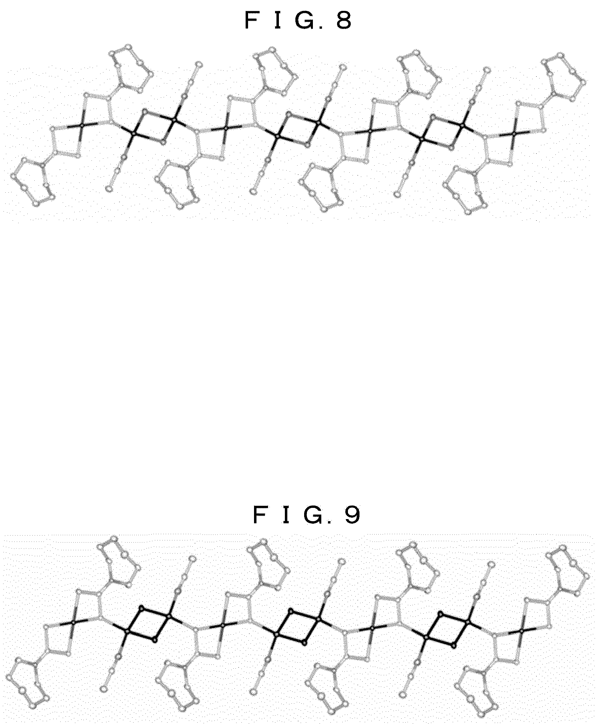

[Fig. 8] Fig. 8 is a schematic view illustrating a three-dimensional structure of a coordination polymer of Production Example 18.
[Fig. 9] Fig. 9 is a schematic view illustrating a three-dimensional structure of a coordination polymer of Production Example 19.

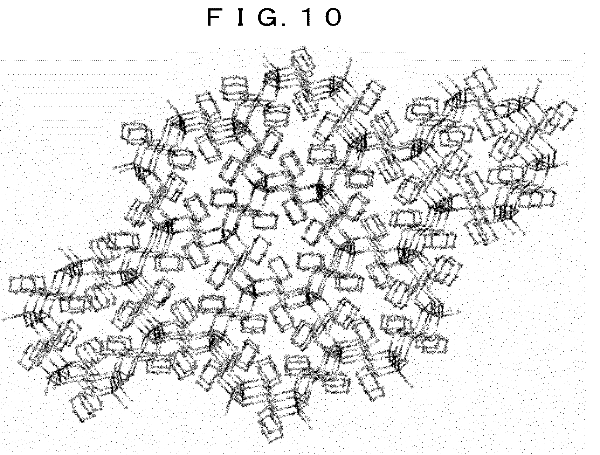

[Fig. 10] Fig. 10 is a schematic view illustrating a three-dimensional structure of a coordination polymer of Production Example 20.

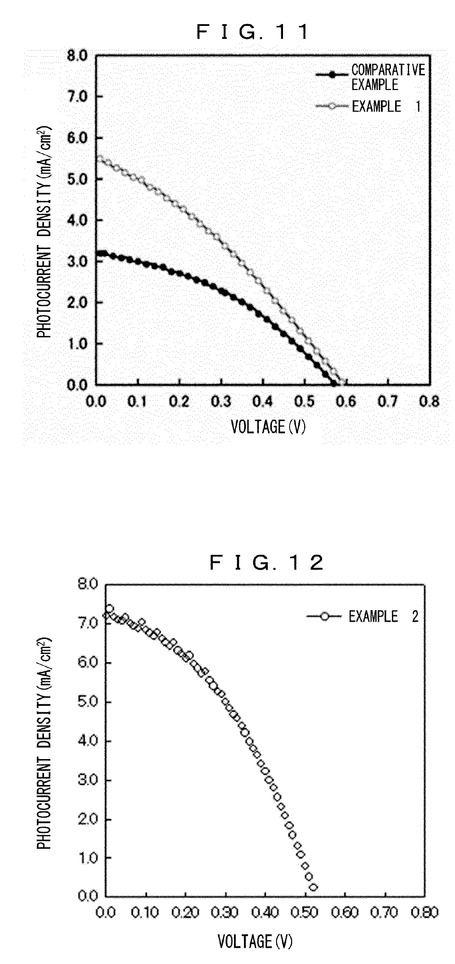

[Fig. 11] Fig. 11 is a graph showing current density-voltage characteristics of a thin-film solar cell of Example 1.
[Fig. 12] Fig. 12 is a graph showing current density-voltage characteristics of a thin-film solar cell of Example 2.
[Fig. 13] Fig. 13 is a graph showing current density-voltage characteristics of a thin-film solar cell of Example 3.
[Fig. 14] Fig. 14 is a schematic view illustrating a coordination polymer having a one-dimensional structure.

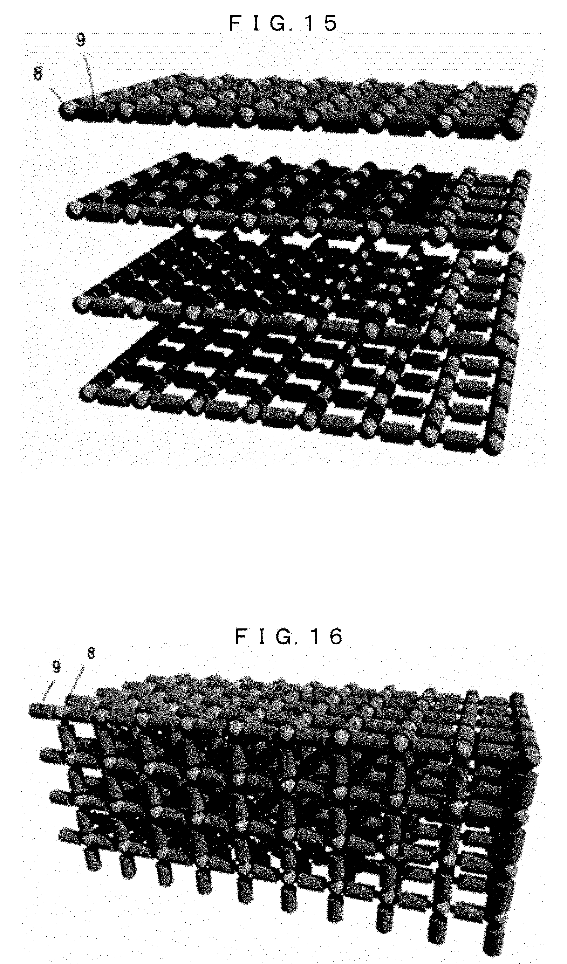

[Fig. 15] Fig. 15 is a schematic view illustrating a coordination polymer having a two-dimensional structure.
[Fig. 16] Fig. 16 is a schematic view illustrating a coordination polymer having a three-dimensional structure. Description of Embodiments

Next, in Production Examples 1 to 7, a detailed description will be made of production examples of a mononuclear complex which is a raw material of a coordination polymer used in a thin-film solar cell of the present invention. It should be understood that the Production Examples described below are only examples that embody the present invention and do not limit the technical scope of the present invention.

### (Production Example 1: Preparation of mononuclear complex Cu(Pip-dtv)₂)

First, 10 mmol of piperidine was added to 100 mL of a methanol solution in which 10 mmol of sodium hydroxide was dissolved, and 10 mmol of carbon disulfide was further allowed to react with the solution.

Next, a solution prepared by dissolving 5 mmol of copper chloride dihydrate in 100 mL of methanol was added to the resulting solution, and allowed to react for five minutes while stirring.

The resulting precipitate was collected by filtration, and then dissolved in 200 mL of chloroform. To the solution, 200 mL of methanol was added, and the resulting solution was concentrated to about 100 mL under reduced pressure. Furthermore, 200 mL of methanol was added thereto, and the resulting solution was concentrated to about 50 mL under reduced pressure. Subsequently, the obtained microcrystals were collected by suction filtration, washed with a small amount of ether, and dried. Thus, a mononuclear complex Cu(Pip-dtc)₂ represented by Chem. 5 was obtained.

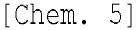

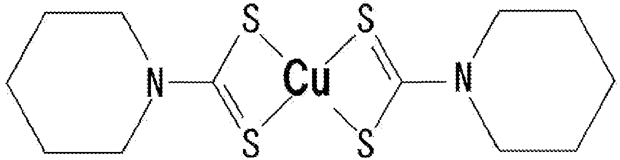

### (Production Example 2: Preparation of mononuclear complex Cu(Hm-dtc)₂)

A mononuclear complex Cu(Hm-dtc)₂ represented by Chem. 6 was obtained as in Production Example 1 except that hexamethyleneimine was used instead of piperidine used in Production Example 1.

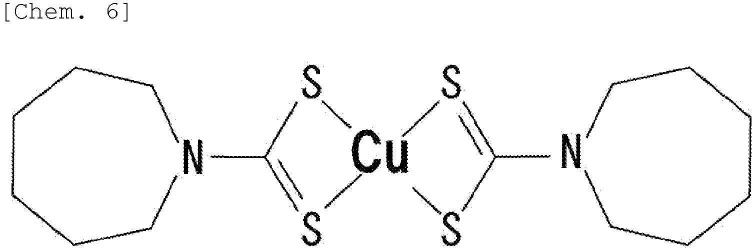

### (Production Example 3: Preparation of mononuclear complex Cu (Pyr-dtc)₂)

A mononuclear complex Cu(Pyr-dtc)₂ represented by Chem. 7 was obtained as in Production Example 1 except that pyrrolizine was used instead of piperidine used in Production Example 1.

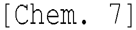

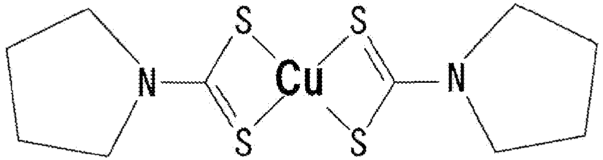

### (Production Example 4: Preparation of mononuclear complex Cu(Ocm-dtc)₂)

A mononuclear complex Cu(Ocm-dtc)₂ represented by Chem. 8 was obtained as in Production Example 1 except that octamethyleneimine was used instead of piperidine used in Production Example 1.

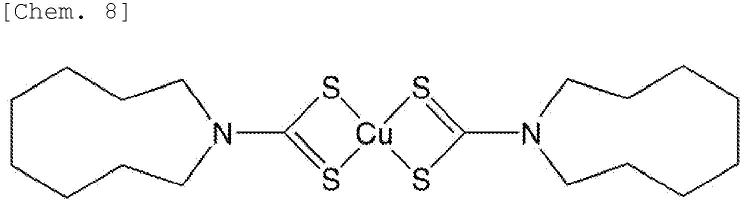

### (Production Example 5: Preparation of mononuclear complex Cu(nPr₂-dtc)₂)

A mononuclear complex Cu(nPr₂-dtc)₂ represented by Chem. 9 was obtained as in Production Example 1 except that dipropylamine was used instead of piperidine used in Production Example 1.

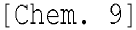

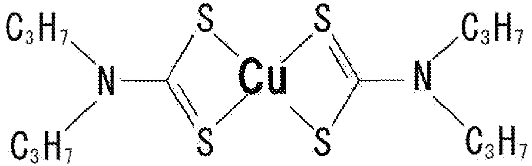

### (Production Example 6: Preparation of mononuclear complex Cu(nBu₂-dtc)₂)

A mononuclear complex Cu(nBu₂-dtc)₂ represented by Chem. 10 was obtained as in Production Example 1 except that dibutylamine was used instead of piperidine used in Production Example 1.

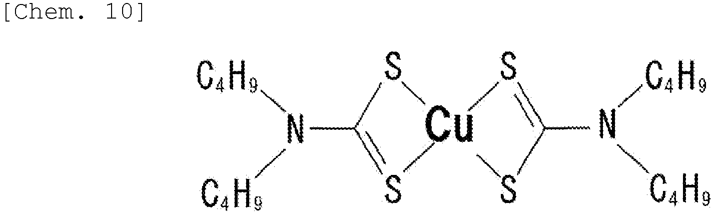

### (Production Example 7: Preparation of mononuclear complex Ni(Pip-dtc)₂)

First, 10 mmol of piperidine was added to 100 mL of a methanol solution in which 10 mmol of sodium hydroxide was dissolved, and 10 mmol of carbon disulfide was further allowed to react with the solution.

Next, a solution prepared by dissolving 5 mmol of nickel chloride hexahydrate in 100 mL of methanol was added to the resulting solution, and allowed to react for five minutes while stirring.

The resulting precipitate was collected by filtration, and then dissolved in 200 mL of chloroform. To the solution, 200 mL of methanol was added, and the resulting solution was concentrated to about 100 mL under reduced pressure. Furthermore, 200 mL of methanol was added thereto, and the resulting solution was concentrated to about 50 mL under reduced pressure. Subsequently, the obtained microcrystals were collected by suction filtration, washed with a small amount of ether, and dried. Thus, a mononuclear complex Ni(Pip-dtc)₂ represented by Chem. 11 was obtained.

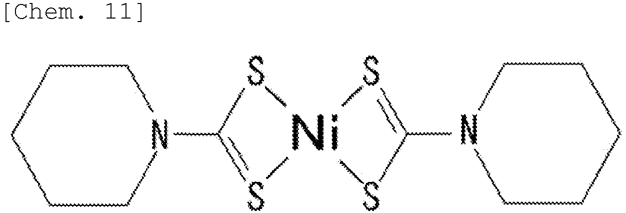

Next, in Production Examples 8 to 22, a detailed description will be made of production examples of a coordination polymer which is a raw material of a thin-film solar cell of the present invention. It should be understood that the Production Examples described below are only examples that embody the present invention and do not limit the technical scope of the present invention.

### (Production Example 8: Preparation of coordination polymer [Cu₅I₃(Pip-dtc)_{4]n})

In 20 mL of chloroform, 0.1 mmol of the mononuclear complex Cu(Pip-dtc)₂ of Production Example 1 was dissolved. In a mixed solvent of 10 mL of propionitrile and 10 mL of acetone, 0.1 mmol of copper iodide was dissolved. These solutions were then mixed, and the mixed solution was left to stand at room temperature for two days. Thus, a coordination polymer ([Cu₅I₃(Pip-dtc)₄]ₙ, black single crystal of (CuIPip1D)) of Production Example 8 was prepared.

Fig. 5 illustrates a three-dimensional structure obtained from a structural analysis of the coordination polymer of Production Example 8. The coordination polymer has a structure in which the mononuclear complex Cu(Pip-dtc)₂ is bonded to both sides of a complex of copper(I) iodide, the complex having a one-dimensional ladder structure.

The coordination polymer of Production Example 8 had a HOMO of -5.09 eV and a LUMO of -3.92 eV. The coordination polymer of Production Example 8 had a conductivity σ_{300K} of 1.1 x 10⁻⁷ S/cm (Eₐ = 0.31 eV).

### (Production Example 9: Preparation of coordination polymer [Cu₃Br₂(Hm-dtc)₂](CH₃CN)₂]ₙ)

In 20 mL of chloroform, 0.1 mmol of the mononuclear complex Cu(Hm-dtc)₂ of Production Example 2 was dissolved. In a mixed solvent of 3 mL of acetonitrile and 17 mL of acetone, 0.2 mmol of copper bromide was dissolved. These solutions were then mixed, and the mixed solution was left to stand at room temperature for one day. Thus, a coordination polymer ([Cu₃Br₂(Hm-dtc)₂(CH₃CN)₂]ₙ, black single crystal of (CuBrHm1D)) of Production Example 9 was prepared.

The coordination polymer of Production Example 9 had a HOMO of -5.20 eV and a LUMO of -3.72 eV. The coordination polymer of Production Example 9 had a conductivity σ_{340K} of 1.7 × 10⁻⁷ S/cm (Eₐ = 0.56 eV).

### (Production Example 10: Preparation of coordination polymer [Cu₃I₂(Hm-dtc)₂](CH₃CN)₂]ₙ)

In 20 mL of chloroform, 0.1 mmol of the mononuclear complex Cu(Hm-dtc)₂ of Production Example 2 was dissolved. In a mixed solvent of 10 mL of acetonitrile and 10 mL of acetone, 0.2 mmol of copper iodide was dissolved. These solutions were then mixed, and the mixed solution was left to stand at room temperature for one day. Thus, a coordination polymer ([Cu₃I₂(Hm-dtc)₂(CH₃CN)₂]ₙ, black single crystal of (CuIHm1D)) of Production Example 10 was prepared.

The coordination polymer of Production Example 10 had a HOMO of -5.10 eV and a LUMO of -3.63 eV. The coordination polymer of Production Example 10 had a conductivity σ_{340K} of 2.46 × 10⁻⁷ S/cm (Eₐ = 0.48 eV).

### (Production Example 11: Preparation of coordination polymer { [Cu₆Br₄ (Pyr-dtc)₄] CHCl₃}ₙ)

In 20 mL of chloroform, 0.1 mmol of the mononuclear complex Cu(Pyr-dtc)₂ of Production Example 3 was dissolved. In 10 mL of acetonitrile, 0.4 mmol of CuBrS(CH₃)₂ was dissolved, and the solution was then diluted with 10 mL of acetone. These solutions were then mixed, and the mixed solution was left to stand at room temperature for one day. Thus, a coordination polymer ({[Cu₆Br₄(Pyr-dtc)₄]CHCl₃}ₙ, black single crystal of (CuBrPyr3D)) of Production Example 11 was prepared.

The coordination polymer of Production Example 11 had a HOMO of -5.28 eV and a LUMO of -4.27 eV. The coordination polymer of Production Example 11 had a conductivity σ_{300K} of 5.2 × 10⁻⁷ S/cm (Eₐ = 0.29 eV).

### (Production Example 12: Preparation of coordination polymer [Cu₃Br₂ (Ocm-dtc) ₂]ₙ)

In 20 mL of chloroform, 0.1 mmol of the mononuclear complex Cu(Ocm-dtc)₂ of Production Example 4 was dissolved. In a mixed solvent of 4 mL of acetonitrile and 16 mL of acetone, 0.2 mmol of copper(I) bromide-dimethyl sulfide complex was dissolved. These solutions were then mixed, and the mixed solution was left to stand at room temperature for three days. Thus, a coordination polymer ([Cu₃Br₂(Ocm-dtc)₂]ₙ, black single crystal of (CuBrOcm1D)) of Production Example 12 illustrated in Chem. 12 was prepared.

The coordination polymer of Production Example 12 had a HOMO of -5.24 eV and a LUMO of -3.96 eV. The coordination polymer of Production Example 12 had a conductivity σ300K of 3.4 × 10⁻⁸ S/cm (Eₐ = 0.39 eV).

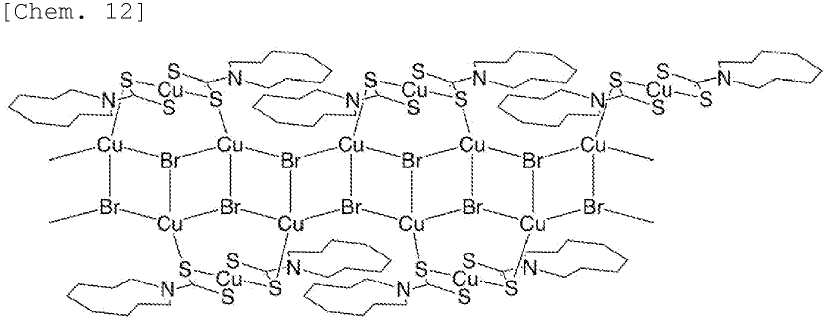

### (Production Example 13: Preparation of coordination polymer [C_{U3}I₂ (Ocm-dtc)₂]ₙ)

In 20 mL of chloroform, 0.1 mmol of the mononuclear complex Cu(Ocm-dtc)₂ of Production Example 4 was dissolved. In a mixed solvent of 10 mL of acetonitrile and 10 mL of acetone, 0.2 mmol of copper(I) iodide was dissolved. These solutions were then mixed, and the mixed solution was left to stand at room temperature for three days. Thus, a coordination polymer ([Cu₃I₂(Ocm-dtc)₂]ₙ, black single crystal of (CuIOcm1D)) of Production Example 13 illustrated in Chem. 13 was prepared.

The coordination polymer of Production Example 13 had a HOMO of -5.19 eV and a LUMO of -3.90 eV. The coordination polymer of Production Example 13 had a conductivity σ_{300K} of 1.1 × 10⁻⁹ S/cm (Eₐ = 0.24 eV).

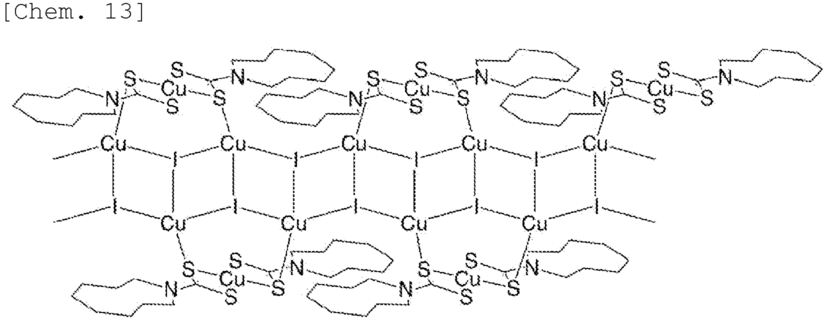

### (Production Example 14: Preparation of coordination polymer [Cu₇Cl₇(nPr₂-dtc)₂]ₙ)

In 20 mL of chloroform, 0.1 mmol of the mononuclear complex Cu(nPr₂-dtc)₂ of Production Example 5 was dissolved. In 20 mL of acetone, 0.4 mmol of copper chloride dihydrate was dissolved. These solutions were then mixed, and the mixed solution was left to stand at room temperature for one day. Thus, a coordination polymer ([Cu₇Cl₇(nPr₂-dtc)₂]ₙ, black single crystal of (CuClnPr2D)) of Production Example 14 was prepared.

The coordination polymer of Production Example 14 had a HOMO of -5.28 eV and a LUMO of -4.76 eV. The coordination polymer of Production Example 14 had a conductivity σ_{300K} of 5.7 × 10⁻⁵ S/cm (Eₐ = 0.21 eV).

### (Production Example 15: Preparation of coordination polymer [Cu₈Br₇(nBu₂-dtc)₂]ₙ)

In 20 mL of chloroform, 0.1 mmol of the mononuclear complex Cu(nBu₂-dtc)₂ of Production Example 6 was dissolved. Next, 0.2 mmol of copper bromide was mixed with a few drops of water, and the resulting mixture was then dissolved in 20 mL of acetone. These solutions were then mixed, and the mixed solution was left to stand at room temperature for one day. Thus, a coordination polymer ([CusBr₇(nBu₂-dtc)₂]ₙ, black single crystal of (CuBrnBu2D)) of Production Example 15 was prepared.

The coordination polymer of Production Example 15 had a HOMO of -5.22 eV and a LUMO of -4.54 eV. The coordination polymer of Production Example 15 had a conductivity σ_{300K} of 2.7 × 10⁻⁵ S/cm (Eₐ = 0.21 eV).

### (Production Example 16: Preparation of coordination polymer [Cu₃Br₂(Pip-dtc)₂(CH₃CN)₂]ₙ)

In 20 mL of chloroform, 0.1 mmol of the mononuclear complex Cu(Pip-dtc)₂ of Production Example 1 was dissolved. In a mixed solvent of 4 mL of acetonitrile and 16 mL of acetone, 0.2 mmol of copper(I) bromide was dissolved. These solutions were then mixed, and the mixed solution was allowed to stand at room temperature for one day. Thus, a coordination polymer ([Cu₃Br₂(Pip-dtc)₂(CH₃CN)₂]ₙ, black single crystal of (α-CuBrPip1D)) of Production Example 16 was prepared. Fig. 6 is a schematic view illustrating a three-dimensional structure of the coordination polymer.

The coordination polymer of Production Example 16 had a HOMO of -5.14 eV and a LUMO of -3.74 eV. The coordination polymer of Production Example 16 had a conductivity σ_{300K} of 3.8 × 10⁻¹⁰ S/cm (Eₐ = 0.66 eV).

### (Production Example 17: Preparation of coordination polymer [Cu₃I₂(Pip-dtc)₂(CH₃CN)₂]ₙ)

In 20 mL of chloroform, 0.1 mmol of the mononuclear complex Cu(Pip-dtc)₂ of Production Example 1 was dissolved. In a mixed solvent of 10 mL of acetonitrile and 10 mL of acetone, 0.1 mmol of copper(I) iodide was dissolved. These solutions were then mixed, and the mixed solution was allowed to stand at room temperature for one day. Thus, a coordination polymer ([C_{U3}I₂(Pip-dtc)₂(CH₃CN)₂]ₙ, black single crystal of (α-CuIPip1D)) of Production Example 17 was prepared. Fig. 7 is a schematic view illustrating a three-dimensional structure of the coordination polymer.

The coordination polymer of Production Example 17 had a HOMO of -5.20 eV and a LUMO of -3.90 eV. The coordination polymer of Production Example 17 had a conductivity σ_{300K} of 4.1 × 10⁻¹⁰ S/cm (Eₐ = 0.50 eV).

### (Production Example 18: Preparation of coordination polymer [Cu₂NiBr₂(Pip-dtc)₂(CH₃CN)₂]ₙ)

In 20 mL of chloroform, 0.1 mmol of the mononuclear complex Ni(Pip-dtc)₂ of Production Example 7 was dissolved. In a mixed solvent of 10 mL of acetonitrile and 10 mL of acetone, 0.2 mmol of copper(I) bromide was dissolved. These solutions were then mixed, and the mixed solution was left to stand at room temperature for one day. Thus, a coordination polymer ([Cu₂NiBr₂(Pip-dtc)₂(CH₃CN)₂]ₙ, black single crystal of (NiBrPip1D)) of Production Example 18 was prepared. Fig. 8 is a schematic view illustrating a three-dimensional structure of the coordination polymer.

The coordination polymer of Production Example 18 had a HOMO of -5.28 eV and a LUMO of -3.90 eV. The coordination polymer of Production Example 18 was an insulator.

### (Production Example 19: Preparation of coordination polymer [Cu₂NiI₂ (Pip-dtc)₂ (CH₃CN)₂]ₙ)

In 20 mL of chloroform, 0.1 mmol of the mononuclear complex Ni(Pip-dtc)₂ of Production Example 7 was dissolved. In a mixed solvent of 10 mL of acetonitrile and 10 mL of acetone, 0.1 mmol of copper(I) iodide was dissolved. These solutions were then mixed, and the mixed solution was left to stand at room temperature for one day. Thus, a coordination polymer ([Cu₂NiI₂(Pip-dtc)₂(CH₃CN)₂]ₙ, black single crystal of (NiIPip1D)) of Production Example 19 was prepared. Fig. 9 is a schematic view illustrating a three-dimensional structure of the coordination polymer.

The coordination polymer of Production Example 19 had a HOMO of -5.22 eV and a LUMO of -3.70 eV. The coordination polymer of Production Example 19 was an insulator.

### (Production Example 20: Preparation of coordination polymer [Cu₂NiBr₂(Pip-dtc)₂]ₙ)

In 20 mL of chloroform, 0.3 mmol of the mononuclear complex Ni(Pip-dtc)₂ of Production Example 7 was dissolved. Next, 0.2 mmol of copper(II) bromide was mixed with a few drops of water, and the resulting mixture was then dissolved in 20 mL of acetone. These solutions were then mixed, and the mixed solution was left to stand at room temperature for one day. Thus, a coordination polymer ([Cu₂NiBr₂(Pip-dtc)₂]ₙ, black block-shaped crystal of (NiBrPip3D)) of Production Example 20 was prepared. Fig. 10 is a schematic view illustrating a three-dimensional structure of the coordination polymer.

The coordination polymer of Production Example 20 had a HOMO of -5.40 eV and a LUMO of -4.39 eV. The coordination polymer of Production Example 20 had a conductivity σ_{300K} of 3.4 × 10⁻¹ S/cm (Eₐ = 0.39 eV).

### (Production Example 21: Preparation of coordination polymer [Cu(dahex-dtc)]ₙ)

In about 500 mL or methanol solvent, 20 mmol of potassium hydroxide was dissolved. Subsequently, 10 mmol of N,N'-diethyl-1,6-diaminohexane, 20 mmol of carbon disulfide, and 10 mmol of copper chloride dihydrate were sequentially added to the solution, thus obtaining a brown precipitate. The brown precipitate was subjected to suction filtration, then dissolved in about 500 mL of chloroform, and filtered. Subsequently, the chloroform was evaporated using an evaporator to some extent, and the resulting precipitate was then diffused with a large amount of hexane. Thus, a coordination polymer (brown precipitate of [Cu(dahex-dtc)]ₙ) of Production Example 21 illustrated in Chem. 14 was prepared.

The coordination polymer of Production Example 21 had a HOMO of -5.05 eV and a LUMO of -3.85 eV. The coordination polymer of Production Example 21 had a conductivity σ_{300K} of 1.2 × 10⁻⁵ S/cm (Eₐ = 0.41 eV).

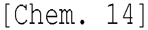

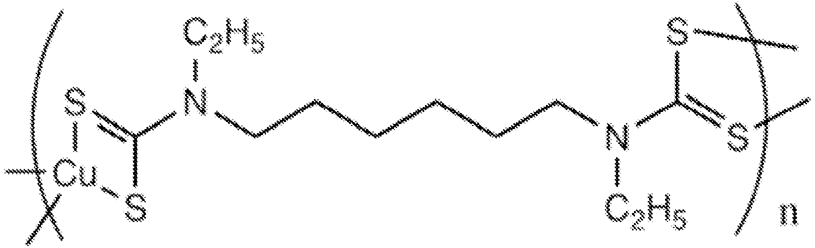

### (Production Example 22: Preparation of coordination polymer [Fe₂(dahex-dtc)₃]ₙ)

In about 500 mL or methanol solvent, 15 mmol of potassium hydroxide was dissolved. Subsequently, 10 mmol of N,N'-diethyl-1,6-diaminohexane, 15 mmol of carbon disulfide, and 10 mmol of iron chloride hexahydrate were sequentially added to the solution, thus obtaining a black precipitate. The black precipitate was subjected to suction filtration, then dissolved in about 500 mL of chloroform, and filtered. Subsequently, the chloroform was evaporated using an evaporator to some extent, and the resulting precipitate was then diffused with a large amount of hexane. Thus, a coordination polymer (black precipitate of [Fe₂(dahex-dtc)₃]ₙ) of Production Example 22 illustrated in Chem. 15 was prepared.

The coordination polymer of Production Example 22 had a HOMO of -4.85 eV and a LUMO of -3.50 eV. The coordination polymer of Production Example 22 had a conductivity σ_{300K} of 2.2 × 10⁻¹¹ S/cm (Eₐ = 0.67 eV).

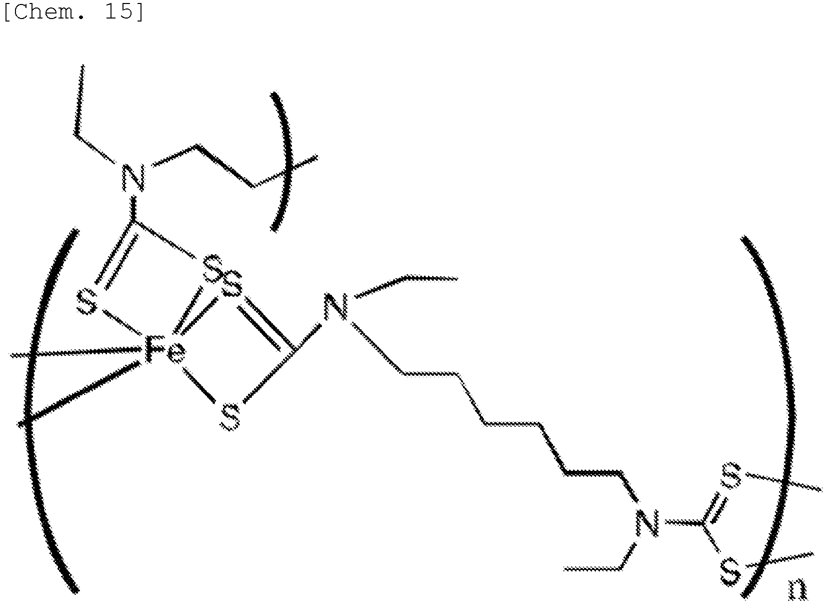

Next, thin-film solar cells of the present invention will be described in detail using Examples and Comparative Example and with reference to the drawings. It should be understood that the Examples described below are only examples that embody the present invention and do not limit the technical scope of the present invention.

### (Example 1: Preparation of thin-film solar cell)

First, poly(3,4-ethylenedioxythiophene) (PEDOT) to which polystyrenesulfonic acid (PSS) was added was applied onto an etched transparent electrode 5 (ITO) using a spin coater at a rotation speed of 2,000 rpm, and dried at 160°C for 10 minutes. This operation was repeated three times to form a PEDOT-PSS film 6 on the transparent electrode 5.

Next, 20 mg of poly-3-hexylthiophene (P3HT), 15 mg of 1-(3-methoxycarbonyl)propyl-1-phenyl-[6,6]-C61 (PCBM), and 0.5 mg of the coordination polymer (CuIPip1D) of Production Example 8 were added to 1 mL of chlorobenzene, and the resulting mixed solution was stirred at 60°C for six hours. Subsequently, the mixed solution was applied onto the PEDOT-PSS film 6 using a spin coater at a rotation speed of 1,000 rpm, and dried at 100°C for 30 minutes.

Furthermore, LiF (0.7 nm) and Al (70 nm) were respectively deposited thereon by vacuum evaporation to form a LiF/Al film 7. Thus, a thin-film solar cell 1 of Example 1 was produced.

Fig. 1 illustrates an example of a cross-sectional structure of the thin-film solar cell 1 of the present invention. It is believed that, unlike a cross-sectional structure of an existing thin-film solar cell which is illustrated in Fig. 2 and in which no coordination polymer is present, the thin-film solar cell 1 of the present invention has a structure in which a coordination polymer 2 is present at an interface between a p-type semiconductor 3 and an n-type semiconductor 4.

Fig. 3 shows a laser microscope photograph of an organic layer of a thin-film solar cell according to the present invention. Unlike a laser microscope photograph of an organic layer of an existing thin-film solar cell illustrated in Fig. 4, fine particles of a coordination polymer having a size from about 1 µm to 10 µm are scattered. This result shows that the coordination polymer 2 is present in the thin film.

### (Example 2: Preparation of thin-film solar cell)

First, poly(3,4-ethylenedioxythiophene) (PEDOT) to which polystyrenesulfonic acid (PSS) was added was applied onto an etched transparent electrode (ITO) using a spin coater at a rotation speed of 2,000 rpm, and dried at 160°C for 10 minutes. This operation was repeated three times to form a PEDOT-PSS film on the transparent electrode.

Next, 20 mg of poly-3-hexylthiophene (P3HT), 15 mg of 1-(3-methoxycarbonyl)propyl-1-phenyl-[6,6]-C61 (PCBM), and 0.5 mg of the coordination polymer (CuBrOcm1D) of Production Example 12 were added to 1 mL of chlorobenzene, and the resulting mixed solution was stirred at 60°C for six hours. Subsequently, the mixed solution was applied onto the PEDOT-PSS film using a spin coater at a rotation speed of 1,000 rpm, and dried at 100°C for 30 minutes.

Furthermore, LiF (0.7 nm) and Al (70 nm) were respectively deposited thereon by vacuum evaporation to form a LiF/Al film. Thus, a thin-film solar cell of Example 2 was produced.

### (Example 3: Preparation of thin-film solar cell)

First, poly(3,4-ethylenedioxythiophene) (PEDOT) to which polystyrenesulfonic acid (PSS) was added was applied onto an etched transparent electrode (ITO) using a spin coater at a rotation speed of 2,000 rpm, and dried at 160°C for 10 minutes. This operation was repeated three times to form a PEDOT-PSS film on the transparent electrode.

Next, 20 mg of poly-3-hexylthiophene (P3HT), 15 mg of 1-(3-methoxycarbonyl)propyl-l-phenyl-[6,6]-C61 (PCBM), and 0.5 mg of the coordination polymer (CuIOcm1D) of Production Example 13 were added to 1 mL of chlorobenzene, and the resulting mixed solution was stirred at 60°C for six hours. Subsequently, the mixed solution was applied onto the PEDOT-PSS film using a spin coater at a rotation speed of 1,000 rpm, and dried at 100°C for 30 minutes.

Furthermore, LiF (0.7 nm) and Al (70 nm) were respectively deposited thereon by vacuum evaporation to form a LiF/Al film. Thus, a thin-film solar cell of Example 3 was produced.

### Comparative Example

A thin-film solar cell of Comparative Example was produced as in Examples except that a mixed solution was prepared without using any of the coordination polymers used in Examples.

Next, current density-voltage characteristics of the thin-film solar cells of Examples 1 to 3 and Comparative Example produced as described above were measured.

### (Measurement of current density-voltage characteristics)

The current density-voltage characteristics were measured using a solar simulator (AM 1.5, 100 mW/cm²). Figs. 11 to 13 show the measurement results.

On the basis of the results shown in Figs. 11 to 13, a current density at a voltage of zero was defined as a short-circuit current density (Jsc), a voltage when a load voltage was applied and the current density became zero was defined as an open-circuit voltage (Voc), a value calculated by dividing the maximum output obtained from a current density-voltage curve by the product of the short-circuit current density and the open-circuit voltage was defined as a fill factor (FF), and a value calculated by dividing the maximum output by the incident light intensity was defined as a conversion efficiency η.

According to the results, the thin-film solar cell of Example 1 had a Jsc of 5.51 mA/cm², a Voc of 0.59 V, an FF of 0.32, and an η of 1.05%. In contrast, the thin-film solar cell of Comparative Example had a Jsc of 3.2 mA/cm², a Voc of 0.58 V, an FF of 0.39, and an η of 0.71%. The thin-film solar cell of Example 2 had a Jsc of 7.204 mA/cm², a Voc of 0.529 V, an FF of 0.396, and an η of 1.508%. The thin-film solar cell of Example 3 had a Jsc of 9.710 mA/cm², a Voc of 0.629 V, an FF of 0.309, and an η of 1.890%.

The above results show that, by using the coordination polymer (Example 1), the short-circuit current density (Jsc) is increased, and consequently, the conversion efficiency η is also improved by 0.34% (48% up), as compared with the case where the coordination polymer is not used (Comparative Example).

### (Measurement of HOMO and LUMO of coordination polymer)

As described in paragraph [0050], the coordination polymer (CuIPip1D) of Production Example 8 used in Example 1 has a HOMO of -5.09 eV and a LUMO of -3.92 eV. Furthermore, it is known that the p-type semiconductor (P3HT) used in Example 1 has a HOMO of -5.0 eV and the n-type semiconductor (PCBM) used in Example 1 has a LUMO of -4.0 eV.

From the relationship of the above energy levels, it is believed that an improvement in the efficiency of the thin-film solar cell of the present invention is caused by the function of the coordination polymer as a sensitizing dye in addition to the known charge separation due to the charge transfer from the p-type semiconductor to the n-type semiconductor. Specifically, first, the coordination polymer absorbs light and causes charge separation to generate electrons and holes. Next, the holes are transported to the HOMO of the p-type semiconductor, whose energy level is higher than the HOMO level of the coordination polymer (CuIPip1D), and the electrons are transported to the LUMO of the n-type semiconductor, whose energy level is lower than the LUMO level of the coordination polymer. It is believed that an electromotive force is generated on the coordination polymer as described above.

Accordingly, it is believed that the short-circuit current density is increased by the sensitizing effect of this additional coordination polymer and the conversion efficiency is improved.

Regarding the coordination polymers other than the coordination polymers prepared in Production Examples 8, 12, and 13, thin-film solar cells including these coordination polymers were not produced. However, the same advantages as those of the thin-film solar cells described in Examples 1 to 3 are achieved by using any of these coordination polymers in combination with a p-type semiconductor having an appropriate HOMO and an n-type semiconductor having an appropriate LUMO.

As described above, according to the thin-film solar cell of the present invention, the conversion efficiency can be improved by selecting an appropriate coordination polymer even in a thin-film solar cell including any organic semiconductor. Thus, the thin-film solar cell of the present invention has a significantly wider range of applications than existing thin-film solar cells including organic semiconductors.

### Industrial Applicability

The present invention can be used in a thin-film solar cell.

### Reference Signs List

- 1: thin-film solar cell
- 2: coordination polymer
- 3: p-type semiconductor
- 4: n-type semiconductor
- 5: transparent electrode
- 6: PEDOT-PSS film
- 7: LiF/Al film
- 8: metal ion
- 9: ligand

## Claims

1. A thin-film solar cell (1) comprising:
at least one organic semiconductor; and
a coordination polymer (2),
wherein the coordination polymer contains
a repeating unit which comprises a complex produced by coordinating at least one ligand (9) to at least one metal ion (8),
the metal ion being selected from ions of transition metal elements, and the ligand being capable of coordinating to the metal ion and selected from sulfur-containing compounds,
nitrogen-containing compounds, oxygen-containing compounds, and phosphorus-containing compounds,
the coordination polymer is
disposed so as to be in contact with a p-type organic semiconductor (3) and an n-type organic semiconductor (4) and to form an interface between the p-type organic semiconductor and the n-type organic semiconductor, and
when two or more organic semiconductors are used as the organic semiconductor,
the two or more organic semiconductors form a bulk-hetero structure,
**characterized in that** the ligand is
at least one derivative selected from derivatives of a dithiocarbamate ion, the derivatives being represented by Chem. 1 and Chem. 2 below: 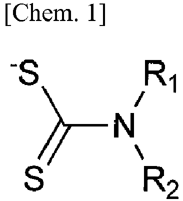 where R₁ and R₂ may be the same or different and each represent an aliphatic hydrocarbon group, a substituted aliphatic hydrocarbon group, an aromatic hydrocarbon group, a substituted aromatic hydrocarbon group, a heterocyclic group, or a substituted heterocyclic group, 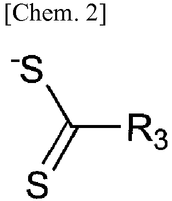 where R₃ represents a heterocyclic group or substituted heterocyclic group having at least one nitrogen atom.

2. The thin-film solar cell according to Claim 1, wherein the transition metal element is
at least one metal element selected from Fe, Co, Ni, Cu, Ru, Rh, Pd, Ag, Os, Ir, Pt, and Au.

3. The thin-film solar cell according to any one of Claims 1 to 2, wherein the coordination polymer further contains
a bromine ion or an iodine ion.

4. The thin-film solar cell according to any one of Claims 1 to 3, wherein the organic semiconductor is
at least one substance selected from fullerene, fullerene derivatives, oligothiophene, oligothiophene derivatives, polythiophene, polythiophene derivatives, phthalocyanine, phthalocyanine derivatives, metal phthalocyanine, metal phthalocyanine derivatives, polyphenylene, polyphenylene derivatives, polyphenylene vinylene, polyphenylene vinylene derivatives, polyvinylcarbazole, polyvinylcarbazole derivatives, polysilane, polysilane derivatives, polyfluorene, polyfluorene derivatives, pentacene, pentacene derivatives, anthracene, anthracene derivatives, rubrene, rubrene derivatives, perylene, perylene derivatives, tetracyanoquinodimethane, tetracyanoquinodimethane derivatives, tetrathiafulvalene, tetrathiafulvalene derivatives, oxadiazole, and oxadiazole derivatives.

5. The thin-film solar cell according to any one of Claims 1 to 4, wherein the organic semiconductor is
1-(3-methoxycarbonyl)propyl-1-phenyl-[6,6]-C61 (PCBM) and poly-3-hexylthiophene (P3HT), and
the coordination polymer contains
copper as the transition metal element, piperidinedithiocarbamic acid as the ligand, and an iodine ion.

## Patentansprüche

1. Dünnschichtsolarzelle (1), umfassend:
mindestens einen organischen Halbleiter; und
ein Koordinationspolymer (2),
wobei das Koordinationspolymer eine Wiederholungseinheit enthält, die einen durch koordinative Anlagerung mindestens eines Liganden (9) an mindestens ein Metall-Ion (8) gebildeten Komplex umfasst,
wobei das Metall-Ion ausgewählt ist aus Ionen von Übergangsmetallelementen, und der Ligand imstande ist, sich an das Metall-Ion koordinativ anzulagern, und ausgewählt ist aus schwefelhaltigen Verbindungen, stickstoffhaltigen Verbindungen, sauerstoffhaltigen Verbindungen und phosphorhaltigen Verbindungen,
das Koordinationspolymer so angeordnet ist, dass es in Kontakt mit einem organischen Halbleiter vom p-Typ (3) und einem organischen Halbleiter vom n-Typ (4) steht und eine Grenzfläche zwischen dem organischen Halbleiter vom p-Typ und dem organischen Halbleiter vom n-Typ bildet, und
wenn zwei oder mehrere organische Halbleiter als organischer Halbleiter verwendet werden, die zwei oder mehreren organischen Halbleiter eine Bulk-Heterostruktur bilden,
**dadurch gekennzeichnet, dass** der Ligand mindestens ein Derivat, ausgewählt aus Derivaten eines Dithiocarbamat-Ions, ist, wobei die Derivate wiedergegeben sind durch nachstehende Formeln Chem. 1 und Chem. 2: 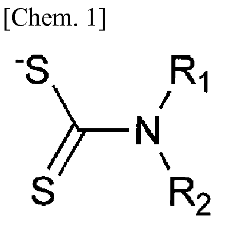
wobei R₁ und R₂ gleich oder verschieden sein können und jeweils einen aliphatischen Kohlenwasserstoffrest, einen substituierten aliphatischen Kohlenwasserstoffrest, einen aromatischen Kohlenwasserstoffrest, einen substituierten aromatischen Kohlenwasserstoffrest, einen heterocyclischen Rest oder einen substituierten heterocyclischen Rest bedeuten, 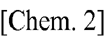 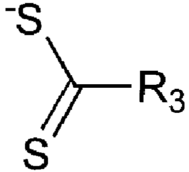 wobei R₃ einen heterocyclischen Rest oder einen substituierten heterocyclischen Rest mit mindestens einem Stickstoffatom bedeutet.

2. Dünnschichtsolarzelle nach Anspruch 1, wobei das Übergangsmetallelement mindestens ein Metallelement, ausgewählt aus Fe, Co, Ni, Cu, Ru, Rh, Pd, Ag, Os, Ir, Pt und Au, ist.

3. Dünnschichtsolarzelle nach einem der Ansprüche 1 bis 2, wobei das Koordinationspolymer weiterhin ein Brom-Ion oder ein Iod-Ion enthält.

4. Dünnschichtsolarzelle nach einem der Ansprüche 1 bis 3, wobei der organische Halbleiter mindestens ein Stoff, ausgewählt aus Fulleren, Fulleren-Derivaten, Oligothiophen, Oligothiophen-Derivaten, Polythiophen, Polythiophen-Derivaten, Phthalocyanin, Phthalocyanin-Derivaten, Metallphthalocyanin, Metallphthalocyanin-Derivaten, Polyphenylen, Polyphenylen-Derivaten, Polyphenylenvinylen, Polyphenylenvinylen-Derivaten, Polyvinylcarbazol, Polyvinylcarbazol-Derivaten, Polysilan, Polysilan-Derivaten, Polyfluoren, Polyfluoren-Derivaten, Pentacen, Pentacen-Derivaten, Anthracen, Anthracen-Derivaten, Rubren, Rubren-Derivaten, Perylen, Perylen-Derivaten, Tetracyanochinodimethan, Tetracyanochinodimethan-Derivaten, Tetrathiafulvalen, Tetrathiafulvalen-Derivaten, Oxadiazol und Oxadiazol-Derivaten, ist.

5. Dünnschichtsolarzelle nach einem der Ansprüche 1 bis 4, wobei der organische Halbleiter 1-(3-Methoxycarbonyl)propyl-1-phenyl-[6,6]-C61 (PCBM) und Poly-3-hexylthiophen (P3HT) ist, und
das Koordinationspolymer Kupfer als Übergangsmetallelement, Piperidindithiocarbamidsäure als Ligand und ein Iod-Ion enthält.

## Revendications

1. Cellule solaire en couches minces (1) comprenant :
au moins un semi-conducteur organique ; et
un polymère de coordination (2),
dans laquelle le polymère de coordination contient
un motif répétitif qui comprend un complexe produit par la coordination d'au moins un ligand (9) à au moins un ion de métal (8),
l'ion de métal étant choisi parmi les ions des éléments métalliques de transition, et le ligand étant capable de se lier par coordination à l'ion de métal et étant choisi parmi les composés contenant du soufre, les composés contenant de l'azote, les composés contenant de l'oxygène et les composés contenant du phosphore,
le polymère de coordination étant
disposé afin d'être en contact avec un semi-conducteur organique de type p (3) et un semi-conducteur organique de type n (4) et de former une interface entre le semi-conducteur organique de type p et le semi-conducteur organique de type n, et
quand deux ou plus de deux semi-conducteurs organiques sont utilisés comme semi-conducteur organique,
les deux ou plus de deux semi-conducteurs organiques forment une hétérostructure volumineuse,
**caractérisée en ce que** le ligand est
au moins un dérivé choisi parmi les dérivés d'un ion dithiocarbamate, les dérivés étant représentés par Chem. 1 et Chem. 2 ci-dessous : 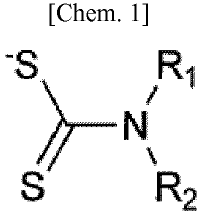 où R₁ et R₂ peuvent être identiques ou différents et représentent chacun un groupe hydrocarboné aliphatique, un groupe hydrocarboné aliphatique substitué, un groupe hydrocarboné aromatique, un groupe hydrocarboné aromatique substitué, un groupe hétérocyclique ou un groupe hétérocyclique substitué, 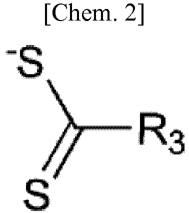 où R₃ représente un groupe hétérocyclique ou un groupe hétérocyclique substitué contenant au moins un atome d'azote.

2. Cellule solaire en couches minces selon la revendication 1, dans laquelle l'élément métallique de transition est au moins un élément métallique choisi parmi Fe, Co, Ni, Cu, Ru, Rh, Pd, Ag, Os, Ir, Pt et Au.

3. Cellule solaire en couches minces selon l'une quelconque des revendications 1 à 2, dans laquelle le polymère de coordination contient en outre
un ion de brome ou un ion d'iode.

4. Cellule solaire en couches minces selon l'une quelconque des revendications 1 à 3, dans laquelle le semi-conducteur organique est
au moins une substance choisie parmi un fullerène, les dérivés de fullerène, un oligothiophène, les dérivés de oligothiophène, un polythiophène, les dérivés de polythiophène, la phtalocyanine, les dérivés de phtalocyanine, une phtalocyanine métallique, les dérivés de phtalocyanine métallique, un polyphénylène, les dérivés de polyphénylène, un polyphénylène vinylène, les dérivés de polyphénylène vinylène, un polyvinylcarbazole, les dérivés de polyvinylcarbazole, un polysilane, les dérivés de polysilane, un polyfluorène, les dérivés de polyfluorène, un pentacène, les dérivés de pentacène, l'anthracène, les dérivés d'anthracène, le rubrène, les dérivés de rubrène, le pérylène, les dérivés de perylène, le tétracyanoquinodiméthane, les dérivés de tétracyanoquinodiméthane, le tétrathiafulvalène, les dérivés de tétrathiafulvalène, l'oxadiazole et les dérivés d'oxadiazole.

5. Cellule solaire en couches minces selon l'une quelconque des revendications 1 à 4, dans laquelle le semi-conducteur organique est
le 1-(3-méthoxycarbonyl)propyl-1-phényl-[6,6]-C61 (PCBM) et le poly-3-hexyl-thiophène (P3HT), et
le polymère de coordination contient
du cuivre en tant qu'élément métallique de transition, de l'acide pipéridinedithiocarbamique en tant que ligand et un ion d'iode.
